# EUROPEAN PATENT APPLICATION

(11) **EP 4 678 142 A1**
(43) Date of publication of application: **14.01.2026**
(21) Application number: 24187972.5
(22) Date of filing: 11.07.2024
(51) Int. Cl.: A61F 2/08, A61B 17/04

(54) **FELTABLE PATCH FOR TISSUE REPAIR HAVING AN AUGMENTING SECTION AND AN ANCHORING SECTION**

(71) Applicant: ZuriMED Technologies AG, 8008 Zürich (CH)
(72) Inventor: SENN, Ronja, 8008 Zürich (CH); ONGINI, Esteban, 8008 Zürich (CH); SCHNYDRIG, Simon, 8008 Zürich (CH); BACHMANN, Elias, 8008 Zürich (CH); LI, Xiang, 8008 Zürich (CH)
(74) Representative: Vossius & Partner Patentanwälte Rechtsanwälte mbB

(57) **Abstract**

The present invention relates to a feltable patch (1, 100) for tissue repair. The patch has an augmenting section (20, 120) and an anchoring section (40, 140). The augmenting section and the anchoring section each comprise or consist of a felt material. The anchoring section and the augmenting section are connected to each other. The anchoring section is convertible from a base configuration to a deployed configuration by a conversion mechanism. The conversion mechanism is configured to expand the anchoring section laterally such that the anchoring section, in the deployed configuration, is configured to anchor the patch in a bone hole.

## Description

The present invention relates to a patch, a set, and a method for anchoring a patch. The present invention may relate to the repair of torn ligaments, tendons and/or intervertebral disc tissue.

Tendon, ligaments, or intervertebral disc tissue may be ruptured, torn partially or completely and can result in need of surgery. The repair procedure can be done with large incisions or with arthroscopic or endoscopic techniques. To repair such structures, the surgeon may use anchors and sutures to reattach the tendon or ligament to the bone. The repaired area may then be covered with a graft to facilitate healing.

Common anchors are often made from a hard material (e.g. a metal), which is fixed in the bone. The anchor is fixed in the bone by a friction fit which is improved by barbs or a similar structure to ensure the firm position of the anchor. However, if such an anchor made from a hard material touches soft tissue due to an inaccuracy during surgery or simply because it moves in the tissue over time, it can lead to irritation or inflammation of the soft tissue in contact with the anchor, and subsequently to an additional surgery.

Often, surgical sutures are used to fix the tendon and tension it to the bone anchor. The fixation of the suture material on the tendon is performed by various stitching techniques. Despite the used stitching technique, this often leads to a suture cut through the retracting tendon and hence to a failure of the repairing construct. A different fixation technique of the soft tissue to the bone anchor is therefore favorable. This patent application builds on the newly introduced technique of surgical felting of a non-woven patch to the tendon, ligament or intervertebral disc tissue.

To fix the sutures to the anchor, various knots can be used. However, tying a knot in a surgical environment, especially in arthroscopic or endoscopic surgeries is a complex and time-consuming process.

In addition, different anatomical structures as well as variations in between different patients require a certain flexibility in implant positioning and final location of the fixation mechanism. Hence, the fixation mechanism should provide a firm fixing of the suture at various distances.

Furthermore, a separation of the different features of an implant to different parts or materials such as anchoring in bone (hard bone anchor), fixation of the tendon (sutures) and facilitating healing (graft) introduces complexity during surgery as well as bears additional risks of failing interfaces.

Finally, among the most urgent soft tissue repair indications is one that completely lacks a current medical solution: intervertebral disk (IVD) annular tears - or in short, IVD tears. IVD tears in the spine are one of the leading causes of long-term disability that cause an enormous healthcare toll. Poor clinical outcomes are reported following IVD repairs with over 30% failure rates due to re-herniation.

Due to limited surgical access to the spine, it is currently not possible to repair IVD tears with conventional surgical stitching techniques. Thus, the state-of-the-art still relies on metal bone fixation that is very invasive, surgically complex, associated with high exposure to radiation and often long-term pain or functional limitations for patients.

US 2018/0296207 A1 discloses a self-punching soft suture anchor insertable into bone without preforming a hole in bone. The suture anchor is formed essentially of two flexible strands. One of the flexible strands has a sinewave configuration. The other flexible strand passes through the length of the flexible strand and circles back. Tightening of this second flexible strand collapses the first flexible strand in an accordion -like fashion.

The present invention addresses one or more of the above problems. In particular, the present invention provides a patch, that improves anchoring, allows for a simpler implementation and/or improves outcomes.

A first aspect of the present invention relates to a feltable patch for tissue repair. The patch has an augmenting section and an anchoring section. The augmenting section and the anchoring section each comprise or consist of a felt material. The anchoring section and the augmenting section are connected to each other. The anchoring section is convertible from a base configuration to a deployed configuration by a conversion mechanism. The conversion mechanism is configured to expand the anchoring section laterally such that the anchoring section, in the deployed configuration, is configured to anchor the patch in a bone hole.

Such a feltable patch is particularly useful for ruptured tendons, ligaments and intervertebral disc tissue as these tissues are exposed to high mechanical forces. The proposed patches can be evenly attached to the damaged tissue avoiding suture cut through using felting and thereby repair the ruptured tissue while at the same time allowing for an additional mechanical fixation to a bone of a patient (using the anchoring section). Thus, the torn tissue is prevented from further damage (e.g. the tear increasing) while at the same time positioning the torn tissue accurately to enable healing. In some embodiments, the patch may additionally comprise a graft, e.g. to enable healing.

Preferably, in the base configuration, the anchoring section and the augmenting section are arranged in a first plane of the patch. The length and width of the patch may be defined in the first plane. The thickness of the patch may be defined perpendicular to the first plane. The patch may be generally rectangular in shape. In the deployed configuration, at least a part of the anchoring section may extend out of the first plane and may form at least one lateral extension extending laterally from the first plane, i.e. in the direction of the thickness of the patch. The anchoring section may form a bulge, or a fold in the deployed configuration. In some examples, the anchoring section may form 2, 3, 4, or more bulges or folds in the deployed configuration. The expansion of the anchoring section may also be described as a bunching up.

The expansion of the anchoring section to the lateral direction causes the anchoring section to be pressed against walls of a bone hole. This provides a secure connection to the bone hole, e.g. with a friction fit. Additionally or alternatively, depending on the shape of the bone hole, a form fit may also be provided. The lateral expansion of the anchoring section may be configured to compress bone tissue below the corticalis (which may be softer than the corticalis). Thereby, the lateral expansion may additionally result in form-fit, blocking the anchoring section underneath the corticalis.

Preferably, the patch is configured for use in a method of preparing or augmenting human or animal soft tissue. Preferably the patch is configured to be used to repair a tendon and/or ligament and/or intervertebral disc tissue. The patch may in particular be configured for a rotator cuff repair or an intervertebral disc repair (e.g. a hernia).

Preferably, the augmenting section is directly connected to the anchoring section and/or the augmenting section and the anchoring section are formed from a single piece of felt material. Thereby, mechanical forces can be efficiently transmitted between the augmenting section and the anchoring section. Especially forming the anchoring section of the augmenting section from a single piece of felt provides for a simple, mechanically stable patch which can be felted to the torn tissue and which is securely anchored using the anchoring section.

The augmenting section, in particular a width of the augmenting section, may transition smoothly into the anchoring section. Transition smoothly may mean without a step, e.g. the width may decrease linearly or along a curve. The transition may be defined along a longitudinal axis of the patch. This may prevent force peaks at steps, at which the patch may begin to fail (e.g. lose its mechanical stability by tearing).

The patch may be for tendon or ligament repair. The patch may measure between 50 mm and 100 mm, preferably between 60 mm and 80 mm in length and/or between 10 mm and 40 mm, preferably between 15 and 30 mm in width. The patch may have a thickness of 0.5 mm to 2.5 mm, preferably 0.8 to 2 mm.

Preferably, at least in the base configuration, a thickness of the patch is limited by an upper surface and an oppositely arranged lower surface. The lower surface is preferably configured for contacting a surface of soft tissue.

Preferably, the felt material is arranged such that some of the fibers of the felt patch can be pushed or pulled through the second surface into soft tissue thereby producing a connection between the felt material and the soft tissue.

Preferably, the patch is for tendon or ligament repair and the augmenting section covers at least 40%, preferably at least 50% of the lower surface of the patch and/or when the anchoring section covers at least 30% and preferably at most 50% of the lower surface of the patch in the base configuration. Ideally, the patch is a small as possible while at the same time providing the required mechanical stability with the anchoring section and covering typical ruptures sufficiently.

Preferably, the anchoring section comprises at least one leg member. The anchoring section may also comprise 2, 3, 4, or more leg members. The leg member extends, in the base configuration from a first connected end of the leg member to a free second end along a longitudinal direction of the leg member. The first end may be connected to the augmenting section. Such leg members may form a particularly simple way to fold up or bunch up the anchoring section. The leg members may be compressed along their longitudinal direction causing the leg member to expand in their lateral direction, i.e. perpendicular to the longitudinal direction of the leg member.

The leg member may have a longitudinal shape. For example, the leg member may have a length that is longer than the width. The leg member is preferably flat in the base configuration. The leg member may have a solid cross-section. The leg member may be formed as a sheet. The leg member may also be a sleeve. For example, the leg member may have a tubular shape.

Preferably, each of the at least one leg members is generally rectangular in shape (i.e. in a top view). The leg member may be sheet-shaped. The leg members may also have a varying width. For example, the width of the leg members may decrease from the connected end to the free end. The width may decrease gradually, or stepwise. For example, the width may decrease linearly at least along a section of the leg member.

While the ends of the leg members are described as connected end and free end, more generally, the ends of the leg members may also be simply described as a first and a second end without necessarily being connected and/or free.

Preferably, a first with the connected end is equal or larger than a second width at the free end. A ratio of the first width and the second width his between 1.0 and 3.0, more preferably between 1.5 and 2.5.

Preferably, each of the at least one leg members has a width, preferably the second width at the free end, of at least 3 mm. More preferably, the second width is at least 4 mm. The first width may be at most 10 mm, preferably at most 8 mm.

Preferably, each of the at least one leg members has, e.g. in the longitudinal direction, a length of at least 20 mm, preferably at least 30 mm and/or of at most 60 mm preferably at most 50 mm. The above measurements may be particular useful for tendon and/or ligament repair.

Preferably, the augmenting section comprises two sides. The two sides may be opposing. The leg members may be provided at the same side of the augmenting section. The leg members may extend in the same direction. For example, a longitudinal direction of the leg members may be parallel. The leg members may be in parallel to each other. Having two leg members at the same side, e.g. extending in parallel, may allow for a more secure attachment as the anchoring section can be attached to two bone holes.

Preferably, the augmenting section comprises two sides and the anchoring section comprises two leg members and the leg members are provided at opposite sides of the augmenting section. Thus, the leg members extend in opposing directions. This may be useful in cases where the patch is intended to bridge a gap between two bone holes, such as intervertebral disc tissue between two vertebrae, wherein a bone whole may be provided in each of two adjacent vertebrae to fixate the anchoring section, i.e. the two leg members extending in opposing directions towards each vertebra, respectively.

Preferably, the deployed configuration, the anchoring section, preferably each of the at least one leg members, forms a bone anchor. Preferably, the deployed configuration, at least a part of the anchoring section is gathered and/or folded such that the at least one lateral extension is formed. The anchor may have the shape of a gathered fabric. The gathered fabric may include one, two, three, four, five or more folds or bulges. The shape of the gathered fabric may be like a flower, a T, or a cloverleaf, in particular when viewed from the side. The conversion mechanism may be a mechanical mechanism. The conversion mechanism may be a pull mechanism. The conversion mechanism may be configured to fold or bunch a first part of the anchoring section at least partly. The conversion mechanism may cause the lateral extension to be formed.

Preferably, the conversion mechanism is provided at the anchoring section, preferably at each of the at least one leg members.

Preferably the conversion mechanism comprises at least one suture. The suture may be fed through the anchoring section. Preferably, at least one suture is fed through each of the at least one leg members. The at least one suture may be fed through the anchoring section, in particular the leg members, in an undulated manner, e.g. in an S-shape or wave form. Thereby, a simple, effective and reliable mechanism for bunching or folding the anchoring section is provided. In one example, at least one suture is fed through each leg member. In another example, one suture is fed through two or more leg members.

Preferably, the patch can be converted from the base configuration to the deployed configuration by actuating the conversion mechanism. In particular, the suture may be pulled. Thereby, the anchoring section may be converted from a generally flat configuration, in particular by pulling the suture, into a three-dimensional configuration, e.g. a flower or cloverleaf shaped configuration. The anchoring section may be at least partly gathered by actuating the conversion mechanism.

Preferably, the anchoring section, in particular one or more of the at least one leg members, comprises a plurality of suture holes. The suture holes define a path of the sutures.

The suture holes on one or more of the at least one leg members may be evenly spaced from each other, in particular equidistantly spaced. The suture holes on one or more of the at least one leg members may be spaced between 3 mm and 8 mm from each other. Thereby, the bunching up causes the anchoring section to securely anchor in bone holes with a size as indicated below.

The spacing of the suture holes may define the size of the folds or bends of the anchoring section. Suture holes that are spaced far apart from each other result in large (but few) bends and suture holes that are close to each other may result in smaller but more numerous bends. The spacing between the suture holes may also be non-uniform. One leg member (or both or more) may comprise two, four, six, eight, or more suture holes. Each additional suture hole may result in one additional bunch or fold. The suture holes may be arranged along a line, preferably a straight line. The straight-line may extend in the respective leg member's longitudinal direction. This may provide for an efficient bunching up. The suture holes may be formed by pushing or pulling the suture through the leg members or the suture holes may be predefined, e.g. with eyelets or using a spike to push aside fibers of the material.

Preferably, the suture holes penetrate through the anchoring section from a lower surface to an upper surface and the suture is fed in a repeated manner from the upper surface through the first one of the holes to a lower surface and through an adjacent one of the holes back to the upper surface. Thereby, the undulating shape of the suture (in the base configuration) is formed.

Preferably, a first end of the suture is fixed to a free end portion of the first leg member and/or wherein the suture extends out of the free end portion of the first leg member. An exit of the deployment sutures at the (free) end of the bone anchors eases the pre-mounting of the implant to the delivery tool. Specifically, it allows better pre-tensioning of the implant on the tool and shortens the required deployment distance for the second bone anchor.

Preferably, a first end of the suture is fixed to the augmentation section or an end of the leg member connected to the augmentation section. The suture may extend out of the augmentation section, or the end of the leg member connected to the augmentation section. This may allow for shorter sutures and less movement of an implantation instrument. The sutures need to be pulled around less, which requires less movement and is advantageous in an arthroscopic environment.

Preferably, the patch is for a tendon or ligament repair and, wherein a lower surface in the plantation section has an area of at least 350 mm², preferably at least 250 mm² and/or at most 1200 mm², preferably at most 1000 mm². These areas (in particular if rectangular or square shaped) may cover typical tears in tendons and ligaments.

Preferably, the augmenting section and/or the lower surface of the augmenting section maybe generally rectangular shaped. More generally, the augmenting section may have a polygon shape, e.g. a triangle, a quadrilateral, a pentagon, or a hexagon. The augmenting section may have a shorter edge length of at least 15 mm, preferably at least 80 mm. A longer edge length may be at least 20 mm, preferably at least 25 mm. The shorter edge length may be measured at a side of the augmenting section. Preferably, the connection between the augmenting section and the anchoring section comprises a cyclic stiffness of at least 25 N/mm, preferably at least 30 N/mm. The cyclic stiffnesses ensure a robust connection and are adapted to typical loads in tendons or ligaments. The cyclic stiffness may be determined as in the protocols described here:
- Christian Jung et al. "Patch-augmented rotator cuff repair: influence of the patch fixation technique on primary biomechanical stability." In: Arch Orthop Trauma Surg (2016).
- Cory O. Nelson et al. "Single-Row Modified Mason-Allen Versus Double-Row Arthroscopic Rotator Cuff Repair: A Biomechanical and Surface Area Comparison." In: Arthroscopy (2008).
- Jeffrey T. Spang. "A Biomechanical Comparison of 2 Transosseous-Equivalent Double-Row Rotator Cuff Repair Techniques Using Bioabsorbable Anchors: Cyclic Loading and Failure Behavior." In: Arthroscopy (2009).

Position measurements of four markers may be taken at time-zero (for details see papers above), after 10 cycles, and at the end of cyclic loading, each at a preload of 10 N to remove potential slack. Gap formation may be defined as the increase in distance (mm) between the medial and lateral markers created during cyclic loading. This parameter may be assessed through the change in position of the markers on the tendon relative to the fixed pins on the humerus. Additionally, as a second measure for the conditioning elongation caused during the 100 cycles, cyclic elongation may be defined as the difference in local maxima between the first and last cycle assessed from the crosshead travel of the material testing machine (Zwick 1456). Peak-to-peak displacement was defined as the average of the local minimum to maximum of the 98th, 99th, and 100th cycles, as described by Ma et al. (Benjamin Ma et al. "Biomechanical Evaluation of Arthroscopic Rotator Cuff Repairs: Double-Row Compared with Single-Row Fixation." In: The Journal of Bone & Joint Surgery (2016)). Cyclic stiffness may be calculated by averaging the stiffness of each cycle determined through the least-square approach. The first cycle of each loading series may not be considered.

The patch, preferably the connection between the augmenting section in the anchoring section, can absorb a maximum force of at least 100 N, preferably at least 150 N.

Preferably, the felt material is biocompatible and/or biodegradable. The felt material may comprise a multitude of fibers. The felt material may comprise fibers made of polyethylene terephthalate, PET, polylactic acid, PLA, polyglutamic acid, PGA, Poly-p-dioxanon, PDS, hyaluronic acid, HA, polydioxanone, PDO, polylactic-co-glycolic acid PLGA, polytetrafluoroethylene, PTFE, polycaprolactone PCL, chitosan, poly-D-lactide, PDLA, poly-DL-lactide, PDLLA, ultra-high-molecular-weight polyethylene, UHMWPE, collagen or poly-L-lactide, PLLA or any combination thereof.

Preferably, the patch is a patch integrated all suture anchor. This may mean that the patch includes the sutures for fixating the patch in place. The patch and/or the felt material of the patch may act as a tendon and/or ligament and/or intervertebral disc tissue augmentation and a bone anchor at once.

Preferably, the felt material comprises fibers. The fibers of the felt may be partially aligned along a longitudinal direction of the patch, in particular a longitudinal direction of at least one of the leg members. Thereby, the tensile strength along the longitudinal direction may be improved without having to add additional material.

Preferably, the anchoring section comprises at least one leg member made of or comprising the felt material. The fibers of the leg member may be arranged parallel to a longitudinal direction of the leg member and/or the augmenting section comprises fibers arranged parallel to a longitudinal direction of the leg member. Preferably, the anchoring section in the augmenting section also comprises fibers parallel to the longitudinal direction.

Preferably, the leg member comprises a first layer and the second layer of felt material. The fibers in the first layer may be arranged parallel to a longitudinal direction of the leg. The fibers of the second layer may be arranged at an angle between 45 and 90° relative to the fibers of the first layer. Thereby a higher resistance of the implant to burst pressure may be achieved. This may be particularly relevant during implantation where forces perpendicular to the sheet are applied to the implant (in particular the anchoring section) to push or hammer the anchoring section into the bone hole. The leg member may comprise a third and/or fourth additional layer. The third layer may include fibers arranged in parallel to the longitudinal direction of the leg member. Further, the second layer may be sandwiched between the first and third layers. The described layers may be in a single leg member. Preferably however, the at least one leg member, i.e. all leg members, and the augmenting section have the described layers.

Preferably, the patch is configured for intervertebral disc repair. Patches for intervertebral disc repair may have the following configuration:
- The patch may measure between 60 and 120 mm, preferably between 70 and 100 mm in length.
- The patch may measure between 5 mm and 30 mm preferably between 8 mm and 20 mm in width.
- The patch may have a thickness of 0.3 mm to 2 mm, preferably 0.5 mm to 1.5 mm.
- The anchoring section may cover at least 15% preferably at least 20% of the lower surface of the patch.
- The anchoring section may cover at most 55% preferably at most 85% of the lower surface of the patch.
- A lower surface of the anchoring section may have an area of at least 60 mm², preferably at least 120 mm².
- A lower surface of the augmenting section may have an area of at most 300 mm², preferably at most 250 mm².
- The augmenting section may have a square shape.
- An edge length of the augmenting section (e.g. of the square shape) may be 8 mm to 20 mm, preferably 11 to 15 mm.

Preferably, the patch comprises a knotless fixation of the conversion mechanism.

The conversion mechanism may comprise a suture configured to expand the anchoring section to the deployed configuration. The suture may be fed through one, two, three, or more eyelets. The first suture section may be proximally to the second suture section (as seen from an operator). The suture is configured to move through the eyelets when the suture is pulled to expand the anchoring section. The suture may be configured to latch to one of the eyelets when pushed in the opposite direction (e.g. against a compression of the anchoring section). This may provide a fixation of the suture without requiring a knot.

A further aspect of the present invention relates to a set comprising a surgical device for attaching a nonwoven textile (e.g. a felt) to human or animal soft tissue. The attachments may comprise pushing individual fibers of the nonwoven textile (e.g. the felt material of the patch) into the human or animal soft tissue. The set may also comprise a patch as described above.

The surgical device may comprise a guide and a felting needle. The felting needle may be arranged movably in the guide. The felting needle may be reciprocally movable between an extended position in which a working tip of the felting needle protrudes beyond an aperture of the device and a retracted position in which the working tip of the felting needle does not protrude beyond the aperture of the device. The guide may be a guide bushing.

The needle may comprise protrusions and or recesses for felting, preferably parts or hooks. Preferably, the needle has a flat tip for pushing individual fibers of the felt material. For example, the felting needle may comprise a flat end section forming a tip of the needle. The flat end section may have a rough surface, protrusions and/or recesses, preferably configured to catch fibers.

Preferably, the needle is configured to move reciprocally with a frequency of at least 20 Hz, preferably of at least 30 Hz and/or of it most 80 Hz, preferably at most 50 Hz.

A further aspect of the present invention relates to a method of anchoring a patch as described above in a bone hole. The method comprises the following steps:
- Guiding at least a part of the anchoring section of the patch through an opening provided in the bone, preferably in the Corticalis, and into the bone;
- Converting the patch from the base configuration into the deployed configuration by actuating the conversion mechanism.

The method may additionally comprise one or more of the following steps:
- Drilling a hole into the bone, wherein the hole preferably penetrates through the Corticalis and wherein the hole forms the opening;
- Placing the anchoring section of the patch in the opening of the bone, e.g. by hammering or pushing the anchoring section into the opening;
- Placing the augmenting section of the patch at a region of soft tissue, preferably across a tear or rupture of the soft tissue;
- Felting the patch, preferably the augmenting section at least partly to the soft tissue, preferably by using the surgical device of the set as described above, wherein the soft tissue preferably is a tendon and/or ligament and/or intervertebral disc tissue and more preferably a tissue or tendon of the rotator cuff and / or tissue of the intervertebral disc.

The opening in the bone may have a depth of at least 10 mm (1 cm) and/or at most 30 mm (3 cm). The opening may have a diameter of at most 3.5 mm, preferably at most 3 mm. Such openings may be particular suitable for tendon or ligament repair.

The opening in the bone may have a depth of at most 25 mm (2.5 cm) and/or a diameter of at most 3 mm, preferably at most 2.8 mm. Such openings may be particular suitable for intervertebral disc repair. The bone may be a bone of a human or another mammal. In some examples the bone may be the humerus or a vertebra.

The step of converting the base configuration into the deployed configuration may include one or more of the following sub-steps:
- expanding at least a part of the anchoring section;
- deforming the patch until the anchoring section extends as wide or wider than the opening;
- expanding at least a part of the anchoring section until the patch is secured in the bone via form fit and/or friction fit; and
- gathering at least a part of the anchoring section.

The method may comprise a step of pulling a suture as described above.

The method may be performed without using any additional anchoring device. In particular the method may be performed, i.e. a patch as described may be implanted, without using additional screws or nails.

A felting may comprise repeatedly advancing and retracting the felting needle through the augmenting section of the patch so that some of the fibers of the patch are pushed or pulled through the low surface into the tissue by means of the felting needle producing a connection between the felt material and the tissue, preferably a tendon and/or ligament and/or intervertebral disc tissue.

The method may be a method of repairing or augmenting human or mammal soft tissue, preferably repairing or augmenting the rotator cuff or repairing the intervertebral disc tissue.

Example embodiments of the present invention are described with reference to the following nonlimiting drawings:
Figure 1 A shows a first embodiment of a patch with two leg members.
Figure 1 B shows a bulging of a leg member.
Figure 2 A shows a second embodiment of a patch with two leg members.
Figure 2 B shows a third embodiment of a patch with two leg members.
Figure 3 A shows a fourth embodiment of a patch with a single leg member.
Figure 3 B shows a fifth embodiment of a patch with two leg members, wherein additionally measurements of the patch are indicated.
Figure 4 A shows a side view of a leg member and a suture for bulging the leg member in a self-locking manner.
Figure 4 B shows an alternative design for self-locking.
Figure 5 shows a further embodiment of a patch.

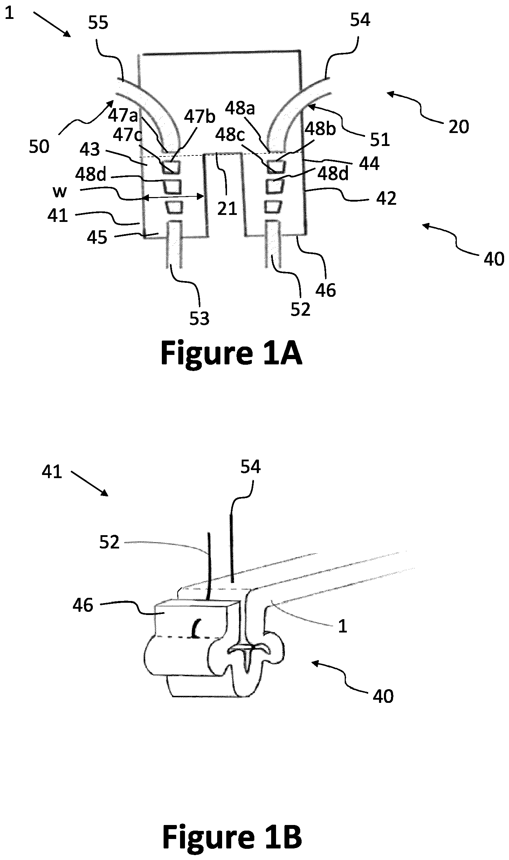

Figure 1 A shows a first example of a patch 1. The patch 1 includes an augmenting section 20 and an anchoring section 40. The augmenting section 20 and/or the anchoring section 40 may each or individually have the shape of a sheet of material. The augmenting section 20 and/or the anchoring section 40 may be made of a felt or felt material. The sheet of material may be flat. In the context of the present specification, the term "flat" may refer to a section (e.g. augmenting section 20 or anchoring section 40) having a length, a width and a thickness perpendicular to the length and the width, wherein a ratio between the length and the thickness and/or a ratio between the width and the thickness is at least 2:1, particularly at least 5:1, more particularly at least 10:1, even more particularly at least 20:1.

The anchoring section 40 and/or the augmenting section 20 may have a thickness as described above. A thickness of the augmenting section 20 may be substantially uniform. A thickness of the anchoring section 40 may also be substantially uniform and may be the same or different as the thickness of the augmenting section.

Preferably, the felt material comprises or consists of fibers made of polyethylene terephthalate, PET, polylactic acid, PLA, polyglutamic acid, PGA, Poly-p-dioxanon, PDS, hyaluronic acid, HA, polydioxanone, PDO, polylactic-co-glycolic acid PLGA, polytetrafluoroethylene, PTFE, polycaprolactone PCL, chitosan, poly-D-lactide, PDLA, poly-DL-lactide, PDLLA, ultra-high-molecular-weight polyethylene, UHMWPE, collagen or poly-L-lactide, PLLA. The fibers of the felt may be made of any of the materials (or any combination thereof) mentioned above.

The felt fibers may have a fiber thickness between 5 and 25 micrometers or 15 to 20 micrometers (e.g. with a tolerance of 5 micrometers). The fibers may have a fiber length of at least 40 or 50 mm (e.g. with a tolerance of 10 mm). In particular, the fiber length may be 50 to 60 mm.

Some of the felt fibers in the augmenting section 20 and/or in the anchoring section 40 are arranged in parallel to a longitudinal direction of at least one leg member 41, 42. Leg members 41, 42 (and optionally additionally the augmenting section 20) may have two (or more) layers of felt material. A first layer may comprise fibers arranged in parallel to a longitudinal direction of at least one leg member. This increases a strength in the longitudinal direction which is particularly advantageous as the mechanical stress is typically applied across the longitudinal direction. A second layer may comprise fibers arranged at an angle between 45° and 90° relative to the fibers of the first layer. This may provide a higher resistance of implant to burst pressure, e.g., when an instrument pushes or hammers the anchoring section into a hole. Additionally, a third layer may be provided.

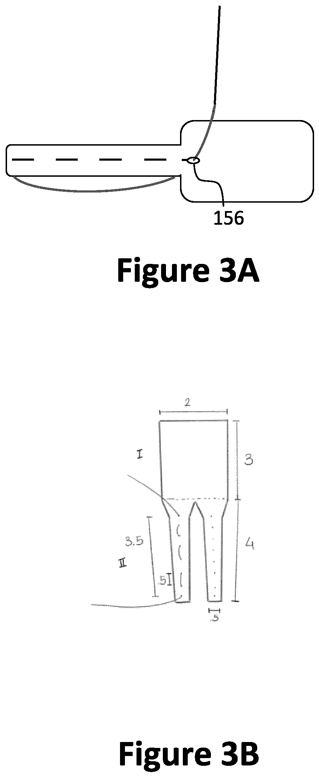

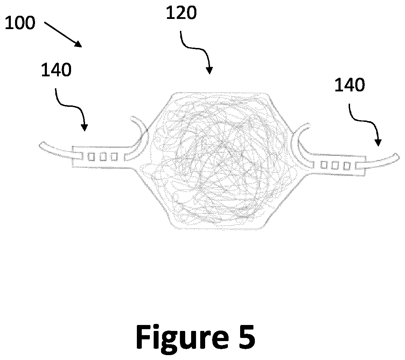

The augmenting section 20 may have a rectangular shape as shown for example in figure 1 A. However, depending on the application, the augmenting section may have other shapes, such as a hexagonal shape as e.g. shown in figure 5, a round shape, a circular shape, a rectangular shape (see e.g. figure 3 A) or otherwise. The augmenting section 20 is suitable to be felted to soft tissue. Example applications will be described in further detail below.

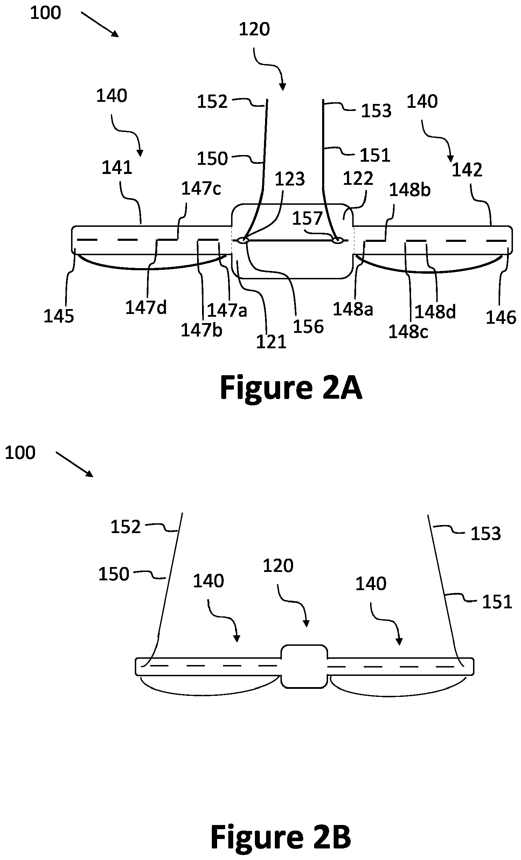

In addition, the patch 1 includes an anchoring section 40 with two leg members 41 and 42. The two leg members 41 and 42 both extend from a first side 21 of the augmenting section 20. The two leg members 41 and 42 extend in the same direction. As will be described in detail below, the two leg members 41 and 42 may extend in the same direction as in figure 1A or may extend in opposite directions as in figures 2A and 2B.

Both leg members 41, 42 may have a rectangular shape as shown in figure 1 A. Preferably, the leg members 41, 42 have a longitudinal direction. This may mean, that a length direction of the leg members 41, 42 is substantially larger (e.g. at least 2 times, 3 times, 5 times or 8 times) larger than a width direction of the leg members 41, 42. In some embodiments, the leg members may form a sleeve made of the sheet shaped felt material. Preferably, the leg members 41, 42 are flat and made of a single sheet of felt material.

The leg members 41, 42 include a connected end 43, 44 that is connected to the augmenting section, and a second, free end 45, 46. In preferred embodiments, the leg members 41, 42 forming the anchoring section 40 and the augmenting section 20 are made from a single sheet of felt material. For example, the shape shown in figure 1 A may be cut from a single sheet of felt material or, fibers (e.g. as described above) may be entangled (i.e. felted to a felt material) and brought into the shape shown in figure 1 A, e.g. by rolling and/or pushing and/or pulling using heat and/or fluids. In other embodiments, the augmenting section 20 and the anchoring section 40 may be made separately from each other and then be connected to each other using conventional techniques (e.g. sutures, tapes, or otherwise).

While the leg members 41, 42 are shown to have a rectangular size in figure 1 A, the leg members 41, 42 may individually or each have a varying width w. For example, the width w may be larger at the connected ends 43, 44 than at the free ends 45, 46. In some examples (see e.g. figure 3B), the leg members 41, 42 may have a conical section in which the width w decreases. The width may decrease gradually or, in other embodiments, stepwise or along a curve.

Further, the leg members 41, 42 include suture holes 47, 48. The suture holes 47, 48 may be cut. Preferably, however, the suture holes 47, 48 may be formed by pushing aside the fibers of the felt. In one example, the sutures 50, 51 may be directly fed through the felt material (e.g. using a needle). In an alternative, the suture holes 47, 48 may be formed by pushing a spike through the anchoring section 40 to form well-defined suture holes 47, 48. Thereby, instead of cutting through the fibers and reducing a mechanical stability of the anchoring section 40, holes are formed without substantial loss in mechanical stability. In some embodiments, the suture holes 47, 48 may be reinforced. For example, the suture holes 47, 48 may be reinforced using eyelets that are fixated in the suture holes. The suture holes 47, 48 are each (or individually, i.e., only suture holes 47 or only suture holes 48) arranged along a straight line. Further, distances between the suture holes 47, 48 on one leg member 41, 42 are evenly spaced. Each leg member 41, 42 (or at least one of the leg members) may include at least two, four, six, or eight suture holes 47, 48. The number of suture holes 47, 48 in one or each leg member 41, 42 may be even.

As can be seen in figure 1A, two sutures 50, 51 are fed through the suture holes 47, 48. Figure 1 A shows a top view of the patch 1. The sutures 50, 51 each (or one of the sutures) enter from a top side through a first suture hole 47a, 48a and transverse the leg members 41, 42 from the top side to a bottom side where the sutures 50, 51 leave the suture holes 47a, 48a. Then, the sutures extend toward the free ends 45, 46 and are fed back from the bottom side to the top side of the patch 1 through second suture holes 47b, 48b. Further, the sutures 50, 51 may be fed again from the top side to the bottom side of the patch 1 through third suture holes 47c, 48c and back to the top side through fourth suture holes 47d, 48d. Similarly, the sutures 50, 51 may be fed back and forth through the patch 1 through four further suture holes. In the example of figure 1A, the leg members each include eight suture holes and thus the sutures 50, 51 are fed four times to the bottom side of the patch 1 and back to the top side of the patch.

Figure 1 A shows the patch 1 in a base configuration, e.g. in a delivery configuration. The patch 1 may be manufactured and packaged in this base configuration or at least be prepared in the base configuration.

The patch 1 may be converted from the base configuration shown in figure 1 A to a deployed configuration shown in figure 1 B. Figure 1B shows the leg member 41 in isolation for illustration purposes. The other leg member 42 may be deployed in the same manner. The suture 50 includes two ends 52 and 54. Similarly, the suture 51 includes two ends 53 and 55. When an operator (e.g., a surgeon, nurse, or other medical professional) holds both ends 52 and 54 and then pulls one of the ends, the leg member 41 is forced to fold or bunch up or bulge as seen in figure 1B. In particular, the leg member 41 expands laterally to the longitudinal direction of the leg member.

This mechanism may be used to anchor the patch 1 in a bone hole. For example, leg member 41 may be pushed or hammered into a bone hole. Then, the operator may pull one or both ends 52, 54 (depending on the fixation of the sutures on the leg member). This expands the anchoring section 40 to the deployed configuration. In particular, the leg member 41 expands laterally pressing the felt material against bony tissue fixating the patch 1 securely in the bone hole. After the leg members 41, 42 were expanded, the sutures 50, 51 may be knotted (e.g. using one or both free ends 52, 54) to lock the anchoring section 40 in the expanded configuration.

The patch 1 shown in figure 1A may be particularly useful for a rotator cuff repair, in particular for tendons and/or ligaments that are torn close to a bony attachment to the bone. In one example, the augmenting section 20 may be felted to the ligament as e.g. described in EP 3 968 899. For example, the augmenting section 20 may cover a partial (or full) tear and hold the torn part of the ligament or tendon together. An example felting device is also disclosed in PCT/EP2024/055924. The felting device described in PCT/EP2024/055924 is incorporated by reference.

The anchoring section 40, i.e. leg members 41, 42 may each be pushed into respective adjacent bone holes and expanded. Thereby, a torn tendon in the rotator cuff (or elsewhere) may be repaired using the described patch 1. The anchoring section 40 provides mechanical support while the augmenting section repairs the torn ligament. The patch 1 is a single integrated device that allows a quick and relatively simple implantation in particular through an arthroscopic access while also providing a reliable and secure attachment. No attachment between different parts of the device within the body is needed.

Example measurements of the device are shown (in cm) in figure 3B. The augmenting section has a width of 2 cm and a length of 3 cm. Each leg member has a length of 4 cm. The free end of the leg members has a width of 0.5 cm. The part of the leg members that is attached to the augmenting section is conically shaped (the width is less towards the free end). The suture holes are distributed over a length of 3.5 cm and may be centered within each leg member. The suture holes are spaced apart by 0.5 cm. Each of these values may be 30%, 20% 10% or 5% larger or smaller.

While the patch 1 may be useful for rotator cuff repair, more generally, the patch 1 may be used to fix other ruptured tendons to bone. Further, while the patch 1 is shown to have an anchoring section with two leg members, the anchoring section may only have a single leg member, e.g. only the leg member 41 (see figure 3A), or more leg members. The single (or both) leg member 41 may be centered with respect to an edge of the augmenting section 20 to which the leg member 41 is attached.

In certain embodiments, the patch 1 may only have leg members extending (at least partially) in one direction, but no leg members extending (at least partially) in the opposite direction.

The present invention may also be used for intervertebral disk (IVD) repair. A tear in an outer, fibrous ring of an intervertebral disc may allow the soft, central portion to bulge out beyond the damaged outer rings. In this case, the disc is said to be herniated.

Example patches 100 for IVD repair are disclosed in figures 2A and 2B. Similarly to the patches for tendon or ligament repair disclosed with reference to figures 1A, 1B and 3B, the patches 100 disclosed in figures 2A and 2B comprise an augmenting section 120, an anchoring section 140 and sutures 150, 151. The augmenting section 120 may be similarly shaped and formed as the augmenting section 20 unless described otherwise. The augmenting section 120 may have a width of 11 mm to 15 mm and a length of 11 mm to 15 mm. The augmenting section 120 may be square shaped as shown in figure 2B or rectangular as shown in figure 2A.

The anchoring section 140 includes a first leg member 141 and a second leg member 142. The leg members 141, 142 are substantially identical to the leg members described with reference to figures 1 A, 1 B and 3 B unless described otherwise. The shown leg members 141, 142 may each (or at least one) have a width of 5 mm and a length of 40 mm.

As can be seen in figures 2 A and 2 B, the leg members 141, 142 are attached to opposing sides 121, 122 of the augmenting section 120. In the particular examples shown in figures 2 A and 2 B, each of the leg members 141, 142 have a longitudinal direction (i.e. the length direction). The leg members 141, 142 are arranged along a common axis. Each of the leg members 141, 142 includes suture holes 147, 148. In the shown example, the leg members 141, 142 each include eight suture holes. In other embodiments, the leg members 141, 142 may include at least 2, at least 4, at least 6, or at least 8 suture holes. As described above, the suture holes may be evenly or unevenly spaced from each other and/or maybe reinforced, e.g. using an eyelet.

Also, the patches 100 may include sutures 150, 151. Additionally to the suture holes 147, 148 in the leg members, the augmenting section 120 may also include a suture hole 123 through which the sutures 150, 151 are each fed. The suture hole in the augmenting section may be more stable and less prone to tears when bulging up the anchoring section. Since forces are highest at the first suture hole, this first suture home may be provided in the augmenting section.

Figures 2 A and 2 B show two alternative configurations for feeding the sutures through the suture holes. In the first configuration shown in figure 2 A, a free end 152, 153 of the sutures (which can be pulled) extends from a suture hole closest to or in the augmenting section 120. In the example shown in figure 2 A, an end of sutures 150, 151 may be fixed at augmenting section 120 or at the connected end of the leg members 141, 142. The end may form an eyelet 156, 157. Then, as described with reference to figure 1A, the sutures 150, 151 traverse the leg members 141, 142 back and forth. Then, the sutures 150, 151 are looped back loosely (e.g. without penetrating the leg member) to the initial fixation of the other end of the suture, at which position it penetrates the patch to extend out of the augmentation section or an end of the leg member connected to the augmentation section, e.g. through eyelets 156, 157 formed at the end fixed to the augmenting section 120 or the connected end of the leg members 141, 142. Further, the example of figure 2 A may allow using a single suture to form sutures 150, 151 extending through all suture holes. In other words, sutures 150, 151 may be made from a single suture. In this case, the sutures 150, 151 may extend in parallel or through the augmenting section 120 and thus further stabilize the augmenting section 120 increasing the ability of the patch 1 to carry loads along its longitudinal direction.

The exit of the free end of the deployment sutures 150, 151 may also be at the connected end of the leg members 141, 142 or in the augmenting section 120 as shown for example in figure 2 A. In this case, the sutures 150, 151 may be shorter. Further, an implantation instrument does not need to move from one end of the patch to another end of the patch (which may be difficult through an arthroscopic access). Since the suture holes 123 from which the free ends of the sutures 150, 151 extend are closer together, an instrument pulling the sutures may not need be moved around as much and there is less risk of dangling sutures entangling with surrounding tissue.

In the second configuration in figure 2 B, a free end 152, 153 of sutures 150, 151 extends from the suture hole in the leg members 141, 142 closest to a free end 145, 146 of the leg members 141, 142. After entering, the sutures 150, 151 feed directly to the augmenting section 120 (e.g. being looped loosely without penetrating the leg member) or to an end of the leg members attached to the augmenting section. Then, as described with reference to figure 1A, the sutures 150, 151 traverse the leg members 141, 142 back and forth. The end of the sutures 150, 151 is fixed to the patch 1, e.g. using a knot at the end of the sutures 150, 151. Preferably, the end of sutures 150, 151 may form an eyelet (similar to figs 2A and 3A, not shown in fig. 2B) through which the sutures 150, 151 extend forming a loop.

An exit of the deployment sutures 150, 151 at the free ends 145, 146 of the anchoring sections (see figure 2B) facilitates the pre-mounting of the implant on the delivery tool. Specifically, it allows better pre-tensioning of the implant on the tool and shortens the required deployment distance for the second bone anchor.

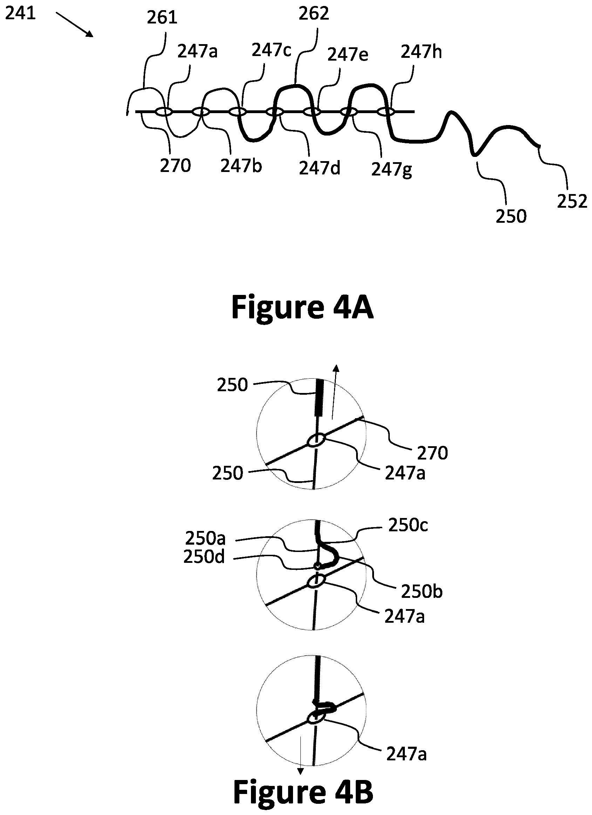

While the sutures 50, 51, 150, 151 may be fixated in the deployed configuration (i.e. laterally expanded) using knots, a knotless fixation may facilitate implantation even further. An example knotless fixation of the sutures 50, 51, 150, 151 is described with reference to figure 4 A. Figure 4 A shows an leg member 241 (e.g. any of the leg members 41, 42, 141, 142) in a side view and illustrates suture holes 247 (e.g. any of the suture holes 47, 48, 147, 148) and a suture 250 (e.g. any of the sutures 50, 51, 150, 151 as shown in figures 1 A to 3B). The suture holes can be formed directly in the felt material, e.g. by reinforcing holes. Preferably, however, the leg member includes a second suture 270 and the second suture 270 includes the plurality of eyelets that form the suture holes 247 through which suture 250 extends.

The suture 250 includes a first section 261 and a second section 262. The first section 261 has a larger cross-section, e.g. a larger diameter, than the second section of the suture 250. In particular, the suture 250 includes a step between the first section 261 and the second section 262. As shown in figure 4 A, the step is within suture hole 247d. A user may grip a free end 252 of the suture 250 and pull to convert the leg member 241 from the shown base configuration to the deployed configuration (see e.g. figure 1 B). When the user pulls the suture 250, the step is moved through the suture holes 247. Once the leg member 241 assumes the deployed configuration, the leg member will be compressed and thus push against the suture 250. The step in the suture 250 may become entangled with one of the suture holes 247 preventing the leg member 241 from leaving the deployed configuration. Thus, the suture 250 can easily be pulled in one direction while preventing release of the leg member (e.g. a ratcheting mechanism). Further, this allows locking at different positions defined by the suture holes 247. This sequence of eyelets can create a ratcheting effect.

An alternative to the ratcheting mechanism disclosed in figure 4 A is shown with respect to figure 4 B. A second possibility to increase the diameter of the second suture is shown in figure 4 B.

Figure 4B shows a single eyelet (e.g. eyelet 247a) of the second suture 270. The first suture 250 may extend through the eyelet 247a (see top part of figure 4B) and can be pulled in a proximal direction to expand the anchoring section laterally. As can be seen in the middle part of figure 4B, the first suture 250 may split up in two different suture wires, i.e. a first suture wire 250a (e.g. a main wire or strand) and a second suture wire 250b (e.g. a loose loop wire or strand). The first and second suture wires 250a, 250b may extend in distal direction from a bifurcation 250c (e.g., the point where the first suture 250 splits into the suture wires 250a, 250b). Distal may mean in a direction away from the operator. The second suture wire 250b includes an opening 250d, through which the first suture wire 250a may extend. An end of the second suture wire 250b may be formed by the opening 250d (see middle part of fig. 4B) or may be loose.

When such a suture 250 is pulled in the proximal direction (see e.g. arrow indicated in top view of figure 4B), the first and second suture wires will be pulled through eyelet 247a as e.g. shown in the top part of figure 4B. When pulled in the proximal direction, the first suture wire 250a and the second suture wire 250b may align (e.g. flatten) and pass through the eyelet. When aligned, the first suture wire 250a and the second suture wire 250b may have the same cross-section as the suture 250 before the split and can easily pass a narrowing (e.g. any of the eyelets 247).

If however, the suture 250 is pushed or pulled in the opposite direction (distal direction, see e.g. arrow indicated in bottom part of figure 4B, distal direction), then the second suture wire 250b is pushed towards the bifurcation 250c, but cannot pass the bifurcation 250c. Rather, the opening of the second suture wire 250b is pushed against the bifurcation 250c preventing the suture from passing through the eyelets 247 (or at least one of them) in the proximal direction (see bottom part of figure 4B).

To fix the tendon to the bone, two different surgical procedures are possible:
- First, the implant is fixed to the tendon or intervertebral disc tissue by surgical felting. As a second step, the bone anchor(s) is(are) pushed or hammered in the bone and as a last step fixed by pulling the deployment suture of the anchor(s). This sequence has the advantage of being able to tension or pull the soft tissue towards the bone.
- As a second possibility, the surgery is started by fixing the bone anchors subsequently in the bone by hammering or pushing them in the bone directly or in previously prepared holes. As a second step, the implant is fixed to the tendon or intervertebral disc tissue by surgical felting. This sequence might be beneficial for an implant presenting two or more bone anchors at opposite ends of the feltable implant portion to tension the feltable implant portion on the defective soft tissue (for example a torn intervertebral disc tissue).

Further aspects of the present disclosure relate to the following items:
1. Feltable patch for tissue repair having an augmenting section and an anchoring section, the augmenting section and the anchoring section each comprising or consisting of a felt material and being connected to each other;
   the anchoring section being convertible from a base configuration to a deployed configuration by a conversion mechanism, wherein
   the conversion mechanism is configured to expand the anchoring section laterally such that the anchoring section, in the deployed configuration, is configured to anchor the patch in a bone hole.
2. Patch according to aspect 1, wherein, in the base configuration, the anchoring section and the augmenting section are arranged in a first plane of the patch, a length and a width of the patch preferably are defined in the first plane and a thickness of the patch preferably is defined perpendicular to the first plane, and the patch preferably is generally rectangular in shape.
3. Patch according to aspect 2, wherein, in the deployed configuration, at least a part of the anchoring section extends out of the first plane and forms at least one lateral extension extending laterally from the first plane.
4. Patch according to any one of the preceding aspects, wherein the patch is configured for use in a method of repairing or augmenting human or animal soft tissue, preferably a tendon and/or ligament and/or intervertebral disc tissue, more preferably wherein the patch is configured for rotator cuff repair or intervertebral disc repair.
5. Patch according to any one of the preceding aspects, wherein the augmenting section is directly connected to the anchoring section and/or wherein the augmenting section and the anchoring section are formed from one single piece of felt material, preferably wherein the augmenting section, in particular a width of the augmenting section, transitions smoothly into the anchoring section.
6. Patch according to any of the preceding aspects, wherein the patch is preferably for a tendon or ligament repair and measures between 50 mm and 100 mm, preferably between 60 mm and 80 mm in length and/or between 10 mm and 40 mm, preferably between 15 mm and 30 mm in width, and/or wherein the patch has a thickness of 0.5 mm to 2.5 mm, preferably of 0.8 to 2 mm.
7. Patch according to any one of the preceding aspects comprising, at least in the base configuration, a thickness of the patch is limited by an upper surface and an oppositely arranged a lower surface, wherein the lower surface preferably is configured for contacting a surface of soft tissue.
8. Patch according to aspect 7, wherein the felt material is arranged such that some of the fibers of the felted patch can be pushed or pulled through the lower surface into soft tissue thereby producing a connection between the felt material and the soft tissue.
9. Patch according to any of aspects 7-8, wherein the patch is preferably for a tendon or ligament repair, and wherein the augmenting section covers at least 40 %, preferably at least 50 % of the lower surface of the patch and/or wherein the anchoring section covers at least 30 % and preferably at most 50 % of the lower surface of the patch.
10. Patch according to any one of the preceding aspects, wherein the anchoring section comprises at least one leg member extending, in the base configuration, from a first connected end being connected to the augmenting section to a free second end in a longitudinal direction.
11. Patch according to aspect 10, wherein, in the base configuration, each of the at least one leg members are preferably generally rectangular in shape.
12. Patch according to aspect 10 or 11, a first width at the connected end is equal or larger than a second width at the second end, preferably wherein a ratio of the first width to the second width is between 1.0 and 3.0, more preferably between 1.5 and 2.5.
13. Patch according to any of aspects 10-12, wherein each of the at least one leg members have a width, preferably a second width, of at least 3 mm, more preferably at least 4 mm, and/or wherein each of the at least one leg member has a width, preferably a first width of at most 10 mm, more preferably at most 8 mm.
14. Patch according to any of aspects 10-13, wherein each of the at least one leg members have, preferably in the longitudinal direction, a length of at least 20 mm, preferably at least 30 mm, and/or of at most 60 mm, preferably at most 50 mm.
15. Patch according to any of aspects 10-14, wherein the augmenting section comprises two sides and the anchoring section comprises two leg members, each of the leg members being provided at the same side of the augmenting section.
16. Patch according to any of aspects 10-15, wherein the augmenting section comprises two sides and the anchoring section comprises two leg members, each of the leg members being provided at opposite sides of the augmenting section.
17. Patch according to any one of the preceding aspects, wherein, in the deployed configuration, the anchoring section, preferably each of the at least one leg members, form an anchor, preferably a bone anchor, wherein the anchor preferably has the shape of a gathered fabric, particularly preferably is shaped like a flower, a T, or cloverleaf, preferably when viewed in the second direction.
18. Patch according to any one of the preceding aspects, wherein, in the deployed configuration, at least a part of the anchoring section is gathered and/or folded such that the at least one lateral extension is formed.
19. Patch according to any one of the preceding aspects, wherein the conversion mechanism is a mechanical mechanism, preferably a pull mechanism and/or wherein the conversion mechanism is configured to fold the first part of the anchoring section at least partly, preferably such that the at least one lateral extension is formed.
20. Patch according to any of the preceding aspects, wherein the conversion mechanism is provided at the anchoring section, preferably at each of the at least one leg members.
21. Patch according to any of the preceding aspects, wherein the conversion mechanism comprises at least one suture, wherein the suture preferably is fed through the anchoring section, more preferably through each of the at least one leg members, particularly preferably in an undulated manner.
22. Patch according to any of the preceding aspects, wherein the patch can be converted from the base configuration to the deployed configuration by actuating the conversion mechanism, preferably by pulling the suture and/or wherein the anchoring section can be converted from a generally flat configuration into a three-dimensional flower or cloverleaf shaped configuration by actuating the conversion mechanism, preferably by pulling the suture, and/or wherein the anchoring section can at least partly be gathered by actuating the conversion mechanism, preferably by pulling the suture.
23. Patch according to aspect 21 or 22, wherein the anchoring section, preferably one or more or each of the at least one leg members comprises a plurality of suture holes.
24. Patch according to aspect 23, wherein the suture holes in one or more leg members are arranged in one generally straight line and preferably spaced between 3 mm and 8 mm from each other, more preferably being equidistantly spaced, wherein the generally straight line, in the base configuration, is preferably defined along the leg member's longitudinal direction.
25. Patch according to aspect 23 or 24, wherein the suture is fed through the holes, preferably wherein the holes penetrate through the anchoring section from a lower surface to an upper surface and the suture is fed in a repeated manner from the upper surface through a first one of the holes to the lower surface and through an adjacent second one of the holes back to the upper surface.
26. Patch according to one of aspects 21 to 25, wherein a first end of the suture is fixed to a free end portion of a first leg member and/or wherein the suture extends out of the free end portion of the first leg member.
27. Patch according to one of aspects 21 to 25, wherein a first end of the suture is fixed to the augmentation section or an end of the leg member connected to the augmentation section and/or wherein the suture extends out of the augmentation section or the end of the leg member connected to the augmentation section.
28. Patch according to any one of the preceding aspects, wherein the patch is preferably for a tendon or ligament repair, and wherein one surface of the augmenting section and/or the lower surface in the augmenting section has an area of at least 350 mm², preferably at least 550 mm² and/or at most 1,200 mm², preferably at most 1,000 mm².
29. Patch according to any one of the preceding aspects, wherein the patch is preferably for a tendon or ligament repair, and wherein wherein the augmenting section and/or the lower surface in the augmenting section is generally rectangular shaped having a shorter edge length of at least 15 mm, preferably at least 18 mm and a longer edge length of at least 20 mm, preferably at least 25 mm, wherein the shorter edge length preferably is measured at a side of the augmenting section.
30. Patch according to any of the preceding aspects, wherein the patch, preferably the connection between the augmenting section and the anchoring section, comprises a cyclic stiffness of at least 25 N/mm, preferably at least 30 N/mm.
31. Patch according to any of the preceding aspects, wherein the patch, preferably the connection between augmenting section and anchoring section, can absorb a maximum force of at least 100 N, preferably at least 150 N.
32. Patch according to any one of the preceding aspects, wherein the felt material is biocompatible and/or biodegradable, wherein the felt material comprises a multitude of fibers and/or wherein the felt material preferably comprises fibers made of polyethylene terephthalate, PET, polylactic acid, PLA, polyglutamic acid, PGA, Poly-p-dioxanon, PDS, hyaluronic acid, HA, polydioxanone, PDO, polylactic-co-glycolic acid PLGA, polytetrafluoroethylene, PTFE, polycaprolactone PCL, chitosan, poly-D-lactide, PDLA, poly-DL-lactide, PDLLA, ultra-high-molecular-weight polyethylene, UHMWPE, collagen or poly-L-lactide, PLLA or any combination thereof.
33. Patch according to any one of the preceding aspects, wherein the patch is a patch integrated all suture anchor, wherein the patch and/or the felt material acts as a tendon and/or ligament and/or intervertebral disc tissue augmentation and a bone anchor at once.
34. Patch according to any one of the preceding aspects, wherein the patch is a graft or implant preferably configured for a direct patch to bone connection.
35. Patch according to one of the preceding aspects, wherein the felt material comprises fibers and wherein the fibers of the felt are at least partially aligned along a longitudinal direction of the patch.
36. Patch according to the preceding aspect, wherein the anchoring section comprises at least one leg member made of or comprising the felt material, and wherein the fibers of the leg member are arranged in parallel to a longitudinal direction of the leg member and/or wherein the augmenting section comprises fibers arranged parallel to a longitudinal direction of the leg member.
37. Patch according to the preceding aspect, wherein the leg member comprises a first layer and a second layer of felt material, wherein the fibers in the first layer are arranged parallel to a longitudinal direction of the leg and wherein the fibers of the second layer are arranged at an angle between 45° and 90° relative to the fibers of the first layer.
38. Patch according to any one of the preceding aspects, wherein the patch is configured for intervertebral disc repair.
39. Patch according to the preceding aspect, wherein the patch measures between 60 mm and 120 mm preferably between 70 mm and 100 mm in length and/or between 5mm and 30 mm, preferably between 8 mm and 20 mm in width, and/or wherein the patch has a thickness of 0.3 mm to 2.0 mm, preferably of 0.5 to 1.5 mm.
40. Patch according to any one of the two preceding aspects, wherein the augmenting section covers at least 15 %, preferably at least 20 % of the lower surface of the patch and/or wherein the anchoring section covers at most 55% and preferably at most 85% of the lower surface of the patch.
41. Patch according to any one of the three preceding aspects, wherein one surface of the augmenting section and/or the lower surface in the augmenting section has an area of at least 60 mm², preferably at least 120 mm² and/or at most 300 mm², preferably at most 250 mm².
42. Patch according to any one of the three preceding aspects, wherein the augmenting section has a square shape, wherein an edge length of the square is preferably 8 mm to 20 mm, more preferably 11 to 15 mm.
43. Patch according to any of the preceding aspects, wherein the patch is configured for a knotless fixation of the conversion mechanism.
44. Patch according to aspect 43, wherein the conversion mechanism comprises a suture configured to expand the anchoring section to the deployed configuration, wherein the suture is fed through one, two, three, four or more eyelets, and wherein the suture is configured to move through the eyelets when the suture is pulled to expand the anchoring section and configured to latch to one of the eyelets when pushed in the opposite direction.
45. Patch according to aspect 44, wherein the suture has a first suture section with a diameter larger than a second suture section, wherein the first suture section is configured to move through the eyelets when the suture is pulled to expand the anchoring section and wherein the first suture section is configured to latch to one of the eyelets when pushed in the opposite direction.
46. Patch according to aspect 44 or 45, wherein the suture includes a section in which the suture splits into a first suture wire and a second suture wire, wherein the second suture wire forms an opening through which the first suture wire extends, wherein the first and second suture wires are configured to move through the eyelets when the suture is pulled to expand the anchoring section and the second suture wire is configured to bulge up against one of the eyelets when the first suture is pushed or pulled in the opposite direction.
47. Set comprising a surgical device for attaching a non-woven textile to human or animal soft tissue by releasing sections of individual fibers of the non-woven textile and carrying them into the human or animal soft tissue, and a patch according to any one of aspects 1-46.
48. Set according to aspect 47, wherein the surgical device comprises a guide and a felting needle being movably arranged in the guide, wherein the felting needle is reciprocally movable between:
   - an extended position in which a working tip of the felting needle protrudes beyond an aperture of the device and
   - a retracted position in which the working tip of the felting needle does not protrude beyond the aperture of the device.
49. Set according to aspect 47 or 48, wherein the felting needle comprises protrusions and/or recesses for felting, preferably barbs or hooks.
50. Set according to aspect 47 or 48, wherein the felting needle comprises an essentially flat end section forming a tip of the needle, wherein the flat end section preferably has a rough surface, protrusions and/or recesses, preferably configured to catch fibers.
51. Set according to any one of aspects 47-50, wherein the needle is configured to reciprocally move with a frequency of at least 20 Hz, preferably of at least 30 Hz and/or of at most 80 Hz, preferably of at most 50 Hz.
52. Method of anchoring a patch according to any of aspects 1-46 in a bone hole, the method comprising the following steps:
   a) Guiding at least a part of the anchoring section of the patch through an opening provided in the bone, preferably in the Corticalis, and into the bone;
   b) Converting the patch from the base configuration into the deployed configuration by actuating the conversion mechanism.
53. Method according to aspect 52, wherein the method further comprises one or more of the following steps:
   c) Drilling a hole into the bone, wherein the hole preferably penetrates through the Corticalis and wherein the hole forms the opening;
   d) Placing the anchoring section of the patch in the opening of the bone;
   e) Placing the augmenting section of the patch at a region of soft tissue;
   f) Felting the patch, preferably the augmenting section at least partly to the soft tissue, preferably by using the surgical device of the set of any of aspects 45-49, wherein the soft tissue preferably is a tendon and/or ligament and/or intervertebral disc tissue and more preferably a tissue or tendon of the rotator cuff and / or tissue of the intervertebral disc.
54. Method according to aspect 51 or 53, wherein the opening in the bone has a depth of at least 10 mm and/or at most 30 mm and/or wherein the opening has a diameter of at most 3.5 mm, preferably at most 3.0 mm, wherein the method is preferably for tendon or ligament repair.
55. Method according to aspect 51 or 53, wherein the opening in the bone has a depth of at most 25 mm and/or wherein the opening has a diameter of at most 3 mm, preferably at most 2.8 mm, wherein the method is preferably for intervertebral disc repair.
56. Method according to any of aspects 51 to 55, wherein step b) comprises one or more of the following:
   - expanding at least a part of the anchoring section;
   - deforming the patch until the anchoring section extends as wide or wider than the opening;
   - expanding at least a part of the anchoring section until the patch is secured in the bone via form fit and/or friction fit;
   - gathering at least a part of the anchoring section.
57. Method according to any of aspects 52 to 56, wherein the bone is a bone of a human or mammal, preferably the Humerus or a vertebra.
58. Method according to any of aspects 52 to 57, wherein the patch is a patch according to any of aspects 20-31 and wherein step b) comprises pulling the suture.
59. Method according to any of aspects 52 to 58, wherein the method is performed without using any additional anchoring devices, preferably without using additional screws or nails.
60. Method according to aspect 59, wherein step f) comprises: repeatedly advancing and retracting a felting needle through the augmenting section of the patch so that some of the fibers of the patch are pushed or pulled through the lower surface into the tissue preferably by means of the felting needle producing a connection between the felt material and the tissue, preferably a tendon and/or ligament and/or intervertebral disc tissue.
61. Method according to any of aspects 52 to 60, wherein the method is a method of repairing or augmenting human or mammal soft tissue, preferably repairing or augmenting the rotor cuff or repairing the intervertebral disc.

## Claims

1. Feltable patch (1, 100) for tissue repair having an augmenting section (20, 120) and an anchoring section (40, 140), the augmenting section (20, 120) and the anchoring section (40, 140) each comprising or consisting of a felt material and being connected to each other;
the anchoring section (40, 140) being convertible from a base configuration to a deployed configuration by a conversion mechanism, wherein
the conversion mechanism is configured to expand the anchoring section (40, 140) laterally such that the anchoring section (40, 140), in the deployed configuration, is configured to anchor the patch (1, 100) in a bone hole.

2. Patch according to claim 1, wherein, in the base configuration, the anchoring section (40, 140) and the augmenting section (20, 120) are arranged in a first plane of the patch (1, 100), a length and a width of the patch (1, 100) preferably are defined in the first plane and a thickness of the patch (1, 100) preferably is defined perpendicular to the first plane, and the patch (1, 100) preferably is generally rectangular in shape.

3. Patch according to claim 2, wherein, in the deployed configuration, at least a part of the anchoring section (40, 140) extends out of the first plane and forms at least one lateral extension extending laterally from the first plane.

4. Patch according to any one of the preceding claims, wherein, in the deployed configuration, at least a part of the anchoring section (40, 140) is gathered and/or folded such that the at least one lateral extension is formed.

5. Patch according to any one of the preceding claims, wherein the patch (1, 100) is configured for use in a method of repairing or augmenting human or animal soft tissue, preferably a tendon and/or ligament and/or intervertebral disc tissue, more preferably wherein the patch (1, 100) is configured for rotator cuff repair or intervertebral disc repair.

6. Patch according to any one of the preceding claims, wherein the anchoring section (40, 140) comprises at least one leg member (41, 42, 141, 142, 241) extending, in the base configuration, from a first connected end being connected to the augmenting section (20, 120) to a free second end in a longitudinal direction.

7. Patch according to claim 6, wherein the augmenting section (20, 120) comprises two opposing sides and the anchoring section (40, 140) comprises two leg members (41, 42, 141, 142), each of the leg members (41, 42, 141, 142) being provided at the same or at opposing sides of the augmenting section (20, 120).

8. Patch according to any of the preceding claims, wherein the conversion mechanism is provided at the anchoring section (40, 140), preferably at each of the at least one leg members (41, 42, 141, 142).

9. Patch according to any of the preceding claims, wherein the patch (1, 100) is configured for a knotless fixation of the conversion mechanism and comprises a ratcheting mechanism that is configured to prevent release of the conversion mechanism.

10. Patch according to one of the preceding claims, wherein the conversion mechanism comprises a suture (50, 51, 150, 151, 250, 270) configured to expand the anchoring section (40, 140) to the deployed configuration, wherein the suture (50, 51, 150, 151, 250, 270) is fed through one, two, three, four or more eyelets (156, 157, 247), and wherein the suture is configured to move through the eyelets (156, 157, 247) when the suture (50, 51, 150, 151, 250, 270) is pulled to expand the anchoring section (40, 140) and configured to latch to one of the eyelets (156, 157, 247) when pushed or pulled in the opposite direction.

11. Patch according to one of the preceding claims, wherein the felt material comprises fibers and wherein the fibers of the felt are at least partially aligned along a longitudinal direction of the patch (1, 100).

12. Patch according to the preceding claim, wherein the anchoring section (40, 140) comprises at least one leg member (41, 42, 141, 142, 241) made of or comprising the felt material, and wherein the fibers of the leg member (41, 42, 141, 142, 241) are arranged in parallel to a longitudinal direction of the leg member (41, 42, 141, 142, 241) and/or wherein the augmenting section comprises fibers arranged parallel to a longitudinal direction of the leg member (41, 42, 141, 142, 241).

13. Patch according to the preceding claim, wherein the leg member (41, 42, 141, 142, 241) comprises a first layer and a second layer of felt material, wherein the fibers in the first layer are arranged parallel to a longitudinal direction of the leg and wherein the fibers of the second layer are arranged at an angle between 45° and 90° relative to the fibers of the first layer.

14. Set comprising a surgical device for attaching a non-woven textile to human or animal soft tissue by releasing sections of individual fibers of the non-woven textile and carrying them into the human or animal soft tissue, and a patch (1, 100) according to any one of the preceding claims.

15. Set according to the preceding claim, wherein the surgical device comprises a guide and a felting needle being movably arranged in the guide, wherein the felting needle is reciprocally movable between:
- an extended position in which a working tip of the felting needle protrudes beyond an aperture of the device and
- a retracted position in which the working tip of the felting needle does not protrude beyond the aperture of the device.
